(19) Europäisches Patentamt
European Patent Office
Office européen des brevets

(11) EP 0 917 872 A1

(12) EUROPEAN PATENT APPLICATION

(43) Date of publication:
26.05.1999 Bulletin 1999/21

(51) Int. Cl.⁶: **A61K 9/00**, A61K 47/48

(21) Application number: 98122202.9

(22) Date of filing: 23.11.1998

(84) Designated Contracting States:
AT BE CH CY DE DK ES FI FR GB GR IE IT LI LU
MC NL PT SE
Designated Extension States:
AL LT LV MK RO SI

(30) Priority: 24.11.1997 KR 9762464

(71) Applicant:
KOREA RESEARCH INSTITUTE OF CHEMICAL
TECHNOLOGY
Daejeon, 305-343 (KR)

(72) Inventors:
• Shin, Byung Chunl
  Daejeon 305-333 (KR)
• Kim, Sung Soo
  Daejeon 305-390 (KR)
• Lee, Byong Min
  Daejeon 305-345 (KR)

(74) Representative:
Weber, Dieter, Dr. et al
Weber, Dieter, Dr.,
Seiffert, Kıaus, Dipl.-Phys.,
Lieke, Winfried, Dr.,
Gustav-Freytag-Strasse 25
65189 Wiesbaden (DE)

(54) **A composition for delivering drug by ionotophoresis**

(57)     The present invention relates to a method for delivering drugs by iontophoresis and more particularly, to a method for improving iontophoresis of effective drugs through skin or mucous membrane, wherein non-ionic or weak ionic effective drug is physically enclosed into carrier, ionic cyclodextrin.

EP 0 917 872 A1

Printed by Xerox (UK) Business Services
2.16.7/3.6

## Description

## BACKGROUND OF THE INVENTION

### Field of the Invention

The present invention relates to a method for delivering drugs by iontophoresis and more particularly, to a method for delivering nonionic or weak ionic effective drug through skin or mucous membrane by improved iontophoresis with physically enclosing the drug into cavity inside of ionic cyclodextrin.

### Description of the Related Art

The iontophoresis refers to a phenomenon that ionic molecule or ion moves from anode to cathode or inversely in accordance with flow of electric current and particularly what is applied to skin or mucous membrane for therapeutics.

A method for delivering drug to skin by iontophoresis was introduced in the 1970s, and methods for delivering ionic drug such as protein and peptide drug became to be presented. The iontophoresis provides driving force which enable ionic drug to be permeated into skin in accordance with electric current. Generally, in order to improve the efficiency of iontophoresis, electrolyte solution as well as ionic drug is required.

A relation between velocity of delivering drug (D) and electric current (I) is expressed by the following Equation 1 according to Faraday's law:

$$D = ItM/ZF \qquad \text{Equation 1}$$

where, M is a molecular weight of drug, Z is an ionized degree, t is partial current rate which is mobilized by drug and may be interpreted as current's efficiency, and F is a Faraday's constant.

According to Equation 1, the velocity of delivering drug (D) depends on electric current (I) in fixed system wherein M, Z and F are fixed and the electric current in drug reservoir is represented by the sum of current density of various ions. The efficiency of delivering drug increases in proportion with current density generated by ionization of effective drug. When electrolyte such as hydrogen ion, metal ion and anion greatly influences on overall current density, the current flow is improved but the efficiency of delivering drug is decreased due to competitive movement between the above ions and effective drug. This is because drug has generally large molecular weight and low electric charge, while electrolyte ion is small and its ionic strength is very strong, which causes large difference of mobility in electric field.

Meanwhile, when the concentration of hydrogen ion in system increases, the adverse effects such as skin stimulus and skin burn are accompanied. The hydrogen ions generated at electrode through pH change due to electrolysis abruptly increase in proportion to the treatment time with electric current. Thus, the removal of ions other than effective drug is a major factor in increasing the efficiency of iontophoresis.

Hitherto, various methods of removing ions other than effective drugs have been disclosed as follows: a) a method of coating electrode with ion-exchange resin or separating electrode and drug reservoir with ion-exchange membrane designed to exclude or remove ions; b) a method of buffing pH change caused by electrolysis with electrolyte associated with immobile macromolecule gel; and c) a method of using Ag/AgCl electrode to inhibit redox reaction caused by electrolysis. However, the above methods are expected only to exclude ions generated at electrode.

Up to now, a method of delivering drug by iontophoresis has been studied as follows: a) a delivery of insulin through pig skin (R. L. Stephev et al., *Biomed. Biochem. Acta,* 43(5), 553(1984)) was performed; b) it was presented through experiment with mouse-rabbit model that the extent of delivering effective drug is larger in pulse current than in direct current (Y. W. Chien, *Int. J. Pharm.,* 44, 197(1988)); and c) electroosmosis-applied delivering method was disclosed with presentation of solvent composition of iontophoresis and delivery system (Korean Patent Appln. No. 88-989 and 88-1245). In addition, the Patents relating to this invention are U.S. Patent No. 4,842,577, 4,878,892, 4,927,408, 4,940,456, 5,003,987 and 5,084,006.

Nevertheless, the above-mentioned methods have still shown low absorption rate and velocity owing to low current efficiency in iontophoresis of nonionic or weak ionic drug, and thus may not exhibit sufficient therapeutic effect.

## SUMMARY OF THE INVENTION

To overcome the aforementioned shortcomings that the conventional methods have faced, the inventor et al. have completed this invention by providing a method for improving the delivery of drugs by iontophoresis performed in such a manner that nonionic or weak ionic effective drug is physically enclosed into ionic cyclodextrin, carrier, and thus prepared enclosure compound exhibits improved permeation to skin by using ionic property of cyclodextrin.

Therefore, an object of this invention is to provide a method for improving the delivery of drug by iontophoresis which employs ionic cyclodextrin as carrier.

### Description of the Drawings

Figure 1 is a graph showing permeation amount of vitamin A by method of this invention and conventional method.

Figure 2 is a graph showing permeation amount of menthol by method of this invention and conventional method.

## Detailed Description of the Invention

The present invention is characterized by a method for delivering drugs by iontophoresis wherein nonionic or weak ionic effective drug is enclosed into ionic cyclodextrin.

This invention is explained in more detail as set forth hereunder:

This invention pertains to a method for improving electrical mobility of effective drugs by iontophoresis caused by ionic property of cyclodextrin, wherein nonionic or weak ionic effective drug is physically enclosed into carrier, ionic cyclodextrin.

This invention employs strong ionic cyclodextrin which is oligosaccharide having 6-12 glucose residues, formed through cleavage of starch by cyclodextrin glucano transferase. The cyclodextrin has unique enclosing capacity, and thus have in recent years raised interest.

The cyclodextrin has ring or donuts form and unique amphipathic character, i.e., its cavity inside with hydrogen atoms shows hydrophobic property while its cavity outside with hydroxyl groups shows hydrophilic property. With the unique amphipathic character of the cyclodextrin, effective drugs such as organic compound is enclosed in its cavity inside, thereby forming enclosure compound with excellent physical character.

The stability of the enclosure compound is mainly dependent on the stereo-compatibility between cyclodextrin as carrier and effective drugs, compound enclosed, and Van der Waals force, hydrogen bond or charge transfer force independently or cooperatively act as bonding force in the enclosure compound.

According to this invention, the typical examples of cyclodextrin include $\alpha$ -cyclodextrin, $\beta$ - cyclodextrin, $\gamma$ - cyclodextrin and derivatives thereof. Also, the cyclodextrin is positive ionic cyclodextrin or negative ionic cyclodextrin; positive ionic group of the positive ionic cyclodextrin is one or more than two ionic groups selected from the group consisting of quadrivalent ammonium, imine and amide group; and negative ionic group of the negative ionic cyclodextrin is one or more than two ionic groups selected from the group consisting of sulfate, carboxylate, phosphate and nitrate group.

The nonionic or weak ionic effective drug employed in this invention is one or more than two therapeutic drugs selected from the group consisting of peptide drug, protein drug, steroid drug, hormone drug, analgesia, antiinflammatory drug, anticancer drug, antiviral drug, antibacterial drug and vitamins.

According to this invention, it is preferred that the effective drugs are of molecular weight in the range of 100-100000; if the molecular weight of the effective drugs is less than 100, the performing iontophoresis becomes meaningless since the drugs very easily permeate through skin or mucous membrane; in the case of exceeding 100000, the iontophoresis is not effective since the drugs hardly permeate through skin or mucous membrane

In this invention, it is preferred that the effective drug is nonionic or weak ionic compound, since cavity inside of cylcodextrin as carrier is hydrophobic as mentioned above and the same or similar electric property is also required for the enclosure compound in order to have an effect in its target where physically enclosed drug is moved to

The enclosing reaction is explained in more detail as follows:

First of all, the very unstable state of the cyclodextrin in terms of energy is required, which is induced by water into hydrophobic cavity inside of cyclodextrin.

The introduction of nonionic or weak ionic effective drug into the cavity inside of the cyclodextrin results in the energetically stable state of the cyclodextrin through pushing out water molecules in the cavity inside and enclosing the drug into the cavity inside. The enclosing reaction is exothermic and thus lowering the reaction temperature causes to move the reaction equilibrium toward formation of enclosure compound.

In an effort to activate the enclosing reaction, the ionic cyclodextrin is mixed and agitated with the effective drug in the presence of water. Then, the enclosure compound with a definite mole ratio is prepared, and the mole ratio depends on the kinds of cyclodextrin and effective drug.

The enclosure compound is prepared by the following methods: a) saturated solution method, b) mixing method and c) drying method, and the typical method, the saturated solution method, is explained as follows:

In the case of saturated solution method, aqueous solution of ionic cyclodextrin is prepared and then effective drugs is slowly added to the solution with agitation, thereby obtaining the enclosure compound. Meanwhile, to obtain the enclosure compound in the form of powder, the above reaction is performed in a manner that the elevated temperature is lowered, thus finally precipitating the stable enclosure compound. Also, with the effective drugs showing low dispersion in oil and water, first of all, the drug is dissolved in suitable solvent having compatibility with water, followed by addition of water to the mixture, and then the enclosing reaction is carried out.

The enclosure compound containing ionic cyclodextrin and effective drugs, so prepared, exhibits several unique functions in the process of iontophoresis as follows:

    a) ionic cyclodextrin of enclosure compound with strong ionic character has driving force that cause the movement of enclosure compound toward opposite electrode to its charge in electric field, and electrolyte which is essential component in iontophoresis is not required since the ionic cyclodextrin is ionized oneself in purified water and thus functions as electrolyte; and

b) the enclosure compound serves as carrier of nonionic drugs, which is seldom carried by conventional iontophoresis, in such a manner that the drug is physically enclosed in ionic cyclodextrin and the enclosure is delivered by driving force due to ionic character of cyclodextrin in electric field. The function is major one of the enclosure compound prepared according to this invention and may be applicable to all nonionic drugs.

The following specific examples are intended to be illustrative of the invention and should not be construed as limiting the scope of the invention as defined by appended claims.

**Example 1. Enclosing reaction with carboxyl methyl cyclodextrin and iontophoresis**

50g of 5% aqueous solution containing carboxyl methyl cyclodextrin was prepared and then animal vitamin A was added to the solution dropwise with vigorous stirring. Alter stirring for 1 hour, the insoluble vitamin A was completely enclosed in carboxyl methyl cyclodextrin and the mixture became transparent, which indicates the enclosing reaction is completed.

The cutaneous permeation test, namely iontophoresis test, was performed by using the enclosure compound with vitamin A as effective drug which was prepared in the above. First of all, the aqueous solution containing the enclosure compound was placed in diffusion cell of 4 m$\ell$. The skin of hairless mouse having the diameter of 15 mm was placed at the middle of the diffusion cell. Then, phosphate buffer solution(pH 7.2) was added to the opposite cell to the diffusion cell, and platinum electrode was immersed in both cell, followed by fixing at sampling port.

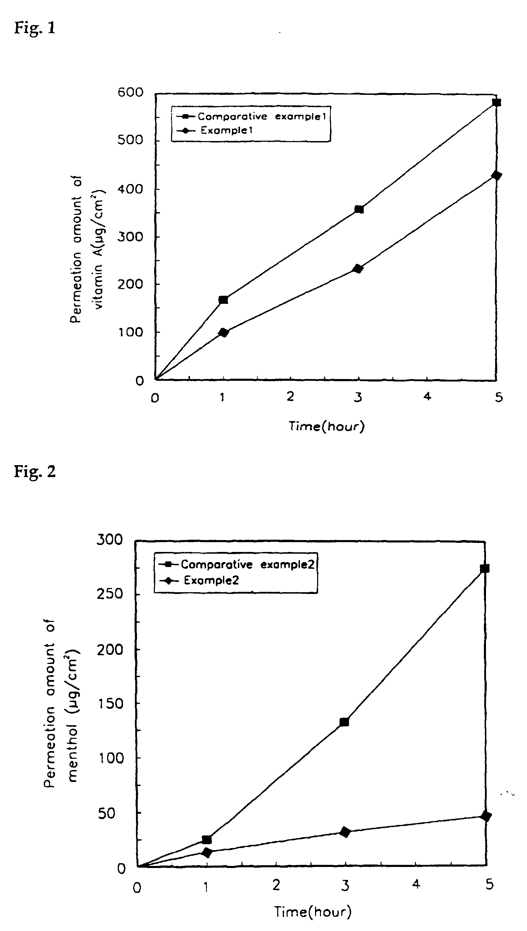

Thereafter, the platinum electrodes were connected to electrical power source to send a constant current of 2 mA until the test was done. And the permeation amount of vitamin A through skin of hairless mouse was measured with HPLC at 352 nm and the results was shown in Figure 1.

**Comparative example 1. Iontophoresis of vitamin A alone**

The vitamin A was dissolved in 50% triethanol amine without enclosing with carboxyl methyl cyclodextrin and the iontophoresis test was performed in the same manner as in Example 1. And the permeation amount of vitamin A through skin of hairless mouse was measured with HPLC at 352 nm and the results was shown in Figure 1.

**Example 2. Enclosing reaction with sulfated cyclodextrin and iontophoresis**

Adding animal menthol and ethanol to 50g of 3% aqueous solution containing sulfated cyclodextrin dropwise with vigorous stirring, the temperature was adjusted at 50 °C. After stirring for 2 hours, the menthol was completely enclosed in sulfated cyclodextrin and the mixture became transparent, which indicates the enclosing reaction is completed. Then, the reaction mixture was stood for more than 1 hour at less than 20 °C filtered or dewatered through centrifugation, and dried at 50 °C.

The cutaneous permeation test, namely iontophoresis test, was performed by using the enclosure compound with menthol as effective drug which was prepared in the above. First of all, the aqueous solution containing the enclosure compound was placed in diffusion cell of 4 m$\ell$. The skin of hairless mouse having the diameter of 15 mm was placed at the middle of the diffusion cell. Then, phosphate buffer solution(pH 7.2) was added to the opposite cell to the diffusion cell, and platinum electrode was immersed in both cell, followed by fixing at sampling port.

Thereafter, the platinum electrode in solution of the enclosure compound was connected to cathode and the platinum electrode in phosphate buffer solution was connected to anode. The electrical power source was connected both electrode to send a constant current of 2 mA until the test was done. And the permeation amount of menthol through skin of hairless mouse was measured with HPLC at 235 nm and the results was shown in Figure 2.

**Comparative example 2. Iontophoresis of menthol alone**

The menthol was dissolved in 50% ethanol solution without enclosing with sulfated cyclodextrin and the iontophoresis test was performed in the same manner as in Example 2. And the permeation amount of menthol through skin of hairless mouse was measured with HPLC at 235 nm and the results was shown in Figure 2.

As mentioned above, it is well understood that the effective drug enclosed in cavity inside of ionic cyclodextrin according to this invention may be effectively improved in terms of permeability to skin due to its improvement of mobility in electric field caused by ionic property of cylodextrin carrier.

**Claims**

1.   A method for delivering drug by iontophoresis characterized in that nonionic or weak ionic effective drug is enclosed into ionic cyclodextrin.

2.   The method for delivering drug by iontophoresis according to claim 1, wherein the ionic cyclodextrin is one or more than two cyclodextrins selected from the group consisting of $\alpha$ -cyclodextrin, $\beta$ - cyclodextrin, $\gamma$ - cyclodextrin and derivatives thereof.

3. The method for delivering drug by iontophoresis according to claim 1 or 2, wherein the ionic cyclodextrin is positive ionic cyclodextrin or negative ionic cyclodextrin.

4. The method for delivering drug by iontophoresis according to claim 3, wherein positive ionic group of the positive ionic cyclodextrin is one or more than two ionic groups selected from the group consisting of quadrivalent ammonium, imine and amide group.

5. The method for delivering drug by iontophoresis according to claim 3, wherein negative ionic group of the negative ionic cyclodextrin is one or more than two ionic groups selected from the group consisting of sulfate, carboxylate, phosphate and nitrate group.

6. The method for delivering drug by iontophoresis according to claim 1, wherein the molecular weight of effective drug is in the range of 100-100000.

7. The method for delivering drug by iontophoresis according to claim 1 or 6, wherein the effective drug is one or more than two therapeutic drugs selected from the group consisting of peptide drug, protein drug, steroid drug, hormone drug, analgesia, anti-inflammatory drug, anticancer drug, antiviral drug, antibacterial drug and vitamins.

Fig. 1

Fig. 2

European Patent
Office

**PARTIAL EUROPEAN SEARCH REPORT**    **Application Number**

which under Rule 45 of the European Patent Convention EP 98 12 2202
shall be considered, for the purposes of subsequent
proceedings, as the European search report

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X | US 5 068 226 A (O'NEILL WILLIAM P ET AL) 26 November 1991 * the whole document * | 1-7 | A61K9/00 A61K47/48 |
| A | PATENT ABSTRACTS OF JAPAN vol. 098, no. 001, 30 January 1998 & JP 09 235230 A (HISAMITSU PHARMACEUT CO INC), 9 September 1997 * abstract * | | |
| A | EP 0 643 981 A (TAKEDA CHEMICAL INDUSTRIES LTD) 22 March 1995 | | |
| A | EP 0 396 184 A (JANSSEN PHARMACEUTICA NV) 7 November 1990 | | |
| A | US 5 476 852 A (CAUWENBERGH GERARD F M J) 19 December 1995 | | |
| A | WO 91 13100 A (AUSTRALIAN COMMERCIAL RESEARCH) 5 September 1991 | | **TECHNICAL FIELDS SEARCHED (Int.Cl.6)** |
| | -/-- | | A61K |

## INCOMPLETE SEARCH

The Search Division considers that the present application. or one or more of its claims, does/do
not comply with the EPC to such an extent that a meaningful search into the state of the art cannot
be carried out, or can only be carried out partially, for these claims

Claims searched completely :

Claims searched incompletely :

Claims not searched :

Reason for the limitation of the search:

see sheet C

| Place of search | Date of completion of the search | Examiner |
|---|---|---|
| THE HAGUE | 11 March 1999 | Fischer, W |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons
 
 & · member of the same patent family, corresponding
document

EPO FORM 1503 03.82 (P04C07)

**PARTIAL EUROPEAN SEARCH REPORT**

| | DOCUMENTS CONSIDERED TO BE RELEVANT | | CLASSIFICATION OF THE APPLICATION (Int.Cl.6) |
|---|---|---|---|
| Category | Citation of document with indication, where appropriate, of relevant passages | Relevant to claim | |
| D,A | R. L. STEPHEN ET AL.: "POTENTIAL NOVEL METHODS FOR INSULIN ADMINISTRATION: I. IONTOPHORESIS"<br>BIOMED. BIOCHIM. ACTA,<br>vol. 43, no. 5, 1984, pages 553-558,<br>XP002096177<br>--- | | |
| D,A | JUE-CHEN LIU ET AL.: "BLOOD GLUCOSE CONTROL IN DIABETIC RATS BY TRANSDERMAL IONTOPHORETIC DELIVERY OF INSULIN"<br>INTERNATIONAL JOURNAL OF PHARMACEUTICS,<br>vol. 44, 1988, XP002096178<br>----- | | |

TECHNICAL FIELDS SEARCHED (Int.Cl.6)

EPO FORM 1503 03.82 (P04C10)

European Patent
Office

**INCOMPLETE SEARCH
SHEET C**

Application Number

EP 98 12 2202

Although claims 1-7 are directed to a method of treatment of the human/animal body (Article 52(4) EPC), the search has been carried out and based on the alleged effects of the compound/composition.

--------------------

Claim(s) searched incompletely:
        1-7

Reason for the limitation of the search (non-patentable invention(s)):

Article 52 (4) EPC - Method for treatment of the human or animal body by therapy

## ANNEX TO THE EUROPEAN SEARCH REPORT
## ON EUROPEAN PATENT APPLICATION NO.

EP 98 12 2202

This annex lists the patent family members relating to the patent documents cited in the above-mentioned European search report.
The members are as contained in the European Patent Office EDP file on
The European Patent Office is in no way liable for these particulars which are merely given for the purpose of information.

11-03-1999

| Patent document cited in search report | | Publication date | Patent family member(s) | | Publication date |
|---|---|---|---|---|---|
| US 5068226 | A | 26-11-1991 | NONE | | |
| EP 0643981 | A | 22-03-1995 | CA 2132574 | A | 23-03-1995 |
| | | | JP 7213627 | A | 15-08-1995 |
| | | | JP 7213628 | A | 15-08-1995 |
| EP 0396184 | A | 07-11-1990 | AT 145136 | T | 15-11-1996 |
| | | | AU 626672 | B | 06-08-1992 |
| | | | AU 5471190 | A | 15-11-1990 |
| | | | CA 2015838 | A | 03-11-1990 |
| | | | DE 69029100 | D | 19-12-1996 |
| | | | DK 396184 | T | 30-12-1996 |
| | | | ES 2096577 | T | 16-03-1997 |
| | | | GR 3022519 | T | 31-05-1997 |
| | | | IE 74192 | B | 16-07-1997 |
| | | | IL 94267 | A | 11-11-1994 |
| | | | JP 2295927 | A | 06-12-1990 |
| | | | JP 2833711 | B | 09-12-1998 |
| | | | PT 93935 | A,B | 08-02-1991 |
| | | | SG 47035 | A | 20-03-1998 |
| | | | US 5476852 | A | 19-12-1995 |
| US 5476852 | A | 19-12-1995 | AT 145136 | T | 15-11-1996 |
| | | | AU 626672 | B | 06-08-1992 |
| | | | AU 5471190 | A | 15-11-1990 |
| | | | CA 2015838 | A | 03-11-1990 |
| | | | DE 69029100 | D | 19-12-1996 |
| | | | DK 396184 | T | 30-12-1996 |
| | | | EP 0396184 | A | 07-11-1990 |
| | | | ES 2096577 | T | 16-03-1997 |
| | | | GR 3022519 | T | 31-05-1997 |
| | | | IE 74192 | B | 16-07-1997 |
| | | | IL 94267 | A | 11-11-1994 |
| | | | JP 2295927 | A | 06-12-1990 |
| | | | JP 2833711 | B | 09-12-1998 |
| | | | PT 93935 | A,B | 08-02-1991 |
| | | | SG 47035 | A | 20-03-1998 |
| WO 9113100 | A | 05-09-1991 | EP 0518930 | A | 23-12-1992 |

EPO FORM P0459

For more details about this annex : see Official Journal of the European Patent Office, No. 12/82